# EUROPEAN PATENT APPLICATION

(11) **EP 1 287 829 A1**
(43) Date of publication of application: **05.03.2003**
(21) Application number: 01120994.7
(22) Date of filing: 31.08.2001
(51) Int. Cl.: A61K 35/78, A61P 9/02, A61P 25/00, A61P 31/00

(54) **An ayurvedic preparation as a sedative, heart tonic compound and as a resistive urinary infection remedy**

(71) Applicant: Sane, Chandrashekhar Hari, Pune-411 030, Maharashtra State (IN)
(72) Inventor: Sane, Chandrashekhar Hari, Pune-411 030, Maharashtra State (IN)
(74) Representative: von Füner, Nicolai, Dr.

(57) **Abstract**

The present invention relates to an ayurvedic preparation as a sedative, a heart toning compound, and as a resistive urinary infection remedy comprising 10 to 80 parts of Balanites aegyptica or its other variation Balanites roxburghii, commonly known as Hingan Bet, and 10 to 90 parts of Caesalpinia sepiaria Roxb (Chilhar), prepared by separately roasting the above two kernel parts of the herbal elementaries at 60 to 80°C, cooling them down, subsequently steeping the components into a liquid extract of tulsi leaves (i.e. decoction) (Ocimum sanctam Linn) and converting them into pulp and mixing together in different proportions as mentioned above, followewd by desiccating the mixture at a temperature of 80°C for three hours to remove the water content therefrom and reducing to powder for preparing tablets or converting into granules for filling in capsules or converting into a injectable medium.

## Description

Present invention relates to an ayurvedic preparation as a sedative, heart toning compound, and as a resistive urinary infection remedy. There are well known preparations/formulations known to ayurveda for controlling several disorders but the age old ayurvedic preparations are pretty specific for particular disorders as such one has to keep a big list of such medicines.

Object of the present invention, therefore, aims at developing an ayurvedic preparation which will be useful to cure number of disorders mainly 6 in numbers. As such, the ayurvedic preparation as per present invention shall simultaneously act as a sedetive, as a heart-toning compound, and as a resistive urinary infection remedy and similar disorders. The inventor desires to name the preparation as Six-in-One. This one ayurvedic preparation, as per present invention, will offer relief and cure nearly six disorders, three of which are mentioned above and other three are related to the same.

The ayurvedic preparation as per present invention will have sedetive effect particularly for the chronic insomaniac patient. This acts as a sedetive and is accomplished by immediately acting upon central nervous system, producing hypnosis. It shall not have any adverse effect as a narcotic. It will help to control the BP and also reduce general muscular pain. Besides, it will also reduce severe headache. It will also act as a mental sedative.

According to another object, the ayurvedic preparation as per present invention aims at controlling palpitation, by acting as a heart toning agent by acting as a sedetive. It is also aimed at offering relief in severe abdominal pain. Further, it is designated to act as a diuretic, thereby helping to reduce oedema. It is also aimed at reducing urinary infection by increasing tissue resistance. The ayurvedic preparation as per present invention is an outcome of prolonged research and actual trials on number of patients over few decades.

According to this invention, therefore, the said ayurvedic preparation is a sedative heart toning compound, and resists urinary infection. It comprises of taking 10 to 80 parts of Balanites aegyptica or its another variation Balanites roxburghii Planch commonly known as Hingan Bet, and 10 to 90 parts of Caesalpinia sepiaria Roxb (Chilhar). These above two kernel parts of the herbal elementaries are taken and separately roasted at 60 to 80 degree centigrade and then cooled down slowly. Thereafter these are steeped in to liquid extract (i.e. decoction) of tulsi leaves (Ocimum sanctam Linn) and are respectively converted into pulp and mixed together in different proportions mentioned as under. The mixture is now desiccated at a temperature of 80 degree centigrade for three hours to remove water content from the same and is reduced to powder for preparing tablets, or converted into granules for filling in capsules or converted as injectable medium. The invention will be further described with the help of examples:

| | | | |
|---|---|---|---|
| Example 1 | a | Hingan Bet | 10% |
| | b | Chilhar | 90% |
| Example 2 | a | Hingan Bet | 25% |
| | b | Chilhar | 75% |
| Example 3 | a | Hingan Bet | 35% |
| | b | Chilhar | 65% |
| Example 4 | a | Hingan Bet | 100% |
| | b | Chilhar | 100% |

Example 5 : The component 'a' 100 parts alone or component 'b' 100 parts alone independently also be processed as explained above.

### SIX-IN-ONE

1. The outer crustation on the grain (Kernal) is separated, and an oxide form (Bhasma) is prepared by usual process by heating, and is mixed in the Kernels (powder) as per the needs of the conditions presented.
2. Caesalpenia ― Sepiaria Roxis, measuring at ½ gm. + Durva (Cynodondactilon) Caesalpenia-Sepiaria Roxismeasuring at 1gm. + Bhasma 1gm. which is finally used for 'Kamla' (jaundice) and even if it can be safely administered as an antacid which works instantly.
3. This crustation ― oxide (Bhasma) will work nicely to remove edimatic conditions (esp. on foot and below the belt) with Caesalpenia ― Sepiaria Roxis measuring in quantity ½ gm. + ½ gm Bhasma.
4. For giddiness Caesalpenia-Sepiaria Roxis 3 doses per day are administered. This also works as nervine tonic ― helps to control blood pressure and induces natural hypnosis. Proportion : Cyperus-Scariosus 2gm + ½ Bhasma + Caesalpenia-Sepiaria Roxis.
5. Caesalpenia-Sepiaria Roxis dose (1gm Plumbago-Zeylahioa) + Vitex-Nirgudi-Trifolia + Cyperus Scariosus controls all abdominal pains ― even if they originate due to Carcinoma, also it resists further growth of this terrible disease. Proportion : 1 gm. Plumbago-Zeylahioa + 1 gm. Vitex-Nirgudi-Trifolia + 1 gm. Cyperus Scariosus + 1gm. Cretoeva Religosa.
6. Caesalpenia-Sepiaria Roxis ½ gram + 3 gm. Gulwel + 1 gm. Ginger (Zinziber-Obbicinate-Rose) + ½ gm Oxide + 3 gms. Gokharu extraction (Tribus-Terrestris) preparation will reinstate natural urination and need not depend on 'Dialysis' and alike remedies.
7. That also works on backache with same proportion with Gokharu (Tribus-Terrestris) added with 1 gm more in proportion for back-ache. This preparation will reinstate natural urination and need not depend on dialysis and alike remedies.
8. Sciatica nerve pain Caesalpenia-Sepiaria Roxis+Gokhru extract (Tribus-Terrestris) + Oxide powder. Proportion: 1gm of Caesalpenia-Sepiaria Roxis + 3 gm. Gokharu (Tribus-Terrestris) + Oxide Powder ½ gm.
9. Caesalpenia-Sepiaria Roxis (1 gm) + Dikemali (Gardenia-Gummifera)- fever which is not controlled by other remedies for the same condition. Proportion : Caesalpenia-Sepiaria Roxis 1 gm + ¼ gm Dikemali (Gardenia-Gummifera).
10. Oedemma of uterus (white discharge) Leucorrhoea. Proportion : Caesalpenia-Sepiaria Roxis + ½ gm + Bhasma ½ gm + 4 gm. Durva (Cynodondactilon).
11. For loss of weight Caesalpenia-Sepiaria Roxis ½ gr. + ¼ gm Bhasma + 2 gm Cyperus-Scariosus + 2 gm Plunbago-Zeylahioa + 2 gms. Cretoeva Religosa.
12. Stone (Urinary-Renal-Calculus) : Tribus-Terrestris 3 gms. + Crotolaria-juncea 3 gms + Curcuma-longa 3 gms. + Quercus-infactoria 5 gms.

## Claims

1. An ayurvedic preparation as a sedative, a heart toning compound, and as a resistive urinary infection remedy comprising 10 to 80 parts of Balanites aegyptica or its other variation Balanites roxburghii, commonly known as Hingan Bet, and 10 to 90 parts of Caesalpinia sepiaria Roxb (Chilhar), prepared by separately roasting the above two kernel parts of the herbal elementaries at 60 to 80°C, cooling them down, subsequently steeping the components into a liquid extract of tulsi leaves (i.e. decoction) (Ocimum sanctam Linn) and converting them into pulp and mixing together in different proportions as mentioned above, followewd by desiccating the mixture at a temperature of 80°C for three hours to remove the water content therefrom and reducing to powder for preparing tablets or converting into granules for filling in capsules or converting into a injectable medium.
